(19) Europäisches Patentamt
European Patent Office
Office européen des brevets

(11) Publication number: **0 223 588 B1**

# (12) EUROPEAN PATENT SPECIFICATION

(45) Date of publication of patent specification: **12.06.91**

(51) Int. Cl.⁵: **C07D 205/08**, C07D 487/04, //(C07D487/04,209:00,205:00)

(21) Application number: **86308977.7**

(22) Date of filing: **18.11.86**

## (54) Process for the preparation of beta-lactam compounds.

(30) Priority: **18.11.85 GB 8528380**

(43) Date of publication of application:
**27.05.87 Bulletin 87/22**

(45) Publication of the grant of the patent:
**12.06.91 Bulletin 91/24**

(84) Designated Contracting States:
**AT BE CH DE ES FR GB GR IT LI LU NL SE**

(56) References cited:
**EP-A-01 066 52**
**EP-A-01 164 32**

**THE JOURNAL OF ORGANIC CHEMISTRY, vol. 50, no. 13, 28th June 1985, pages 2327-2331, American Chemical Society; T. KAMETANI et al.: "Chiral synthesis of the key intermediates of (+)- and (-)-thienamycin"**

**CHEMICAL ABSTRACTS, vol. 105, no. 17, 27th October 1986, page 667, abstract no. 152772c, Columbus, Ohio, US; D.K. DUTTA et al.: "A facile synthesis of functionalized novel monocyclic trans-beta-lactams"**

(73) Proprietor: **IMPERIAL CHEMICAL INDUSTRIES PLC**
**Imperial Chemical House, Millbank**
**London SW1P 3JF(GB)**

(72) Inventor: **Gravestock, Michael Barry**
**72 Moss Lane**
**Bramhall, Stockport, Cheshire(GB)**

(74) Representative: **Denerley, Paul Millington, Dr. et al**
**Imperial Chemical Industries PLC Legal Department: Patents P.O. Box 6, Bessemer Road**
**Welwyn Garden City Hertfordshire AL7 1HD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 223 588 B1

## Description

The invention relates to a process for the manufacture of intermediates useful in the synthesis of beta-lactam antibiotics, particularly carbapenem compounds.

In this specification formulae denoted by roman numerals are set out on separate sheets. Numbered groups eg R1, R2 etc used in more than one formula are to be taken as having the same meaning throughout the specification unless otherwise stated.

Compounds of the formula I

(wherein $R^1$, $R^3$, $R^4$ and $R^5$ have a range of values known in the literature and of which examples will be given hereinafter) and salts and esters thereof, are known to have antibiotic activity.

The invention provides a process for the manufacture of beta-lactam compounds of formula II (which are useful as intermediates in the preparation of compounds of formula I) wherein R2 is a (1-4C)alkyl group optionally substituted by one or more hydroxy groups (optionally protected) or halogen atoms or $R^2$ is a group $R^2$a which is a precursor of such a group wherein $R^2$a is an acyl group or a protected form thereof or is an alkenyl group;

$R^6$ is

(i) $-C(R^3)(R^4)X$ ;

(ii) $-C(R^3)(R^4)CH_2X$ ;

(iii) $-C(R^3)(R^4)COCH_2X$ ;

(iv) -COOH or -COSH ;

(v) $-C(R^3)(ZR^a)X$ ;

wherein $R^3$ and $R^4$ are independently hydrogen or (1-4C)alkyl, $R^a$ is (1-4C)alkyl or phenyl, X is selected from -COOH, -CHO and $-CH_2OH$ and Z is oxygen, sulphur or selenium (wherein in any of the above groups $R^6$ any carboxyl, thiocarboxyl, oxo or hydroxy groups may be protected); and

$R^7$ represents hydrogen, an amino protecting group or a group $-C(R^3)(R^4)COOH$ or a protected form thereof:

which process includes the step of reacting a metal alkyne of the formula III with a nitrone of the formula IV wherein M is a metal ion.

The preferred metal alkyne of formula III is a copper (I) alkyne. Compounds formed with other metal ions may also be useful in the process of the invention and the invention extends to the use of such compounds.

A metal alkyne of formula III may exist in polymeric form under the conditions used for the process of the invention. Although the compound of formula III is represented herein for convenience as a discrete monomer it will be understood that the invention extends to the use of such polymeric forms.

The compound of formula III may be prepared for example by reaction of an alkyne of formula V with the appropriate metal ion in solution. Suitable solvents for this reaction include polar protic solvents for example alcohols, eg methanol and ethanol, or polar aprotic solvents for example dimethylsulphoxide (DMSO) and dimethylformamide (DMF). Appropriate mixtures of solvents may be used.

The metal ion may advantageously be provided in the form of a soluble complex thereof, for example a complex with ammonia or an amine, eg diethanolamine or pyridine. Alternatively, certain solvents, eg DMSO and DMF, may act both as solvents and as complexing agents.

Thus a convenient method of forming the metal alkyne of formula III is to react the metal ion, in the form of a salt soluble in the chosen solvent, with the complexing agent and the alkyne of formula V. The reagents may be added to the solvent in any order. In certain cases it may be useful to pre-dissolve the alkyne in a solvent (which may be one of those already mentioned, or any other compatible solvent, eg water). Where the metal ion is copper (I), a suitable soluble salt is a halide, particularly the chloride.

The reaction is usually complete in a short time, eg 30 min to 2 h, and takes place conveniently at room temperature, although heating or cooling may be employed to regulate the reaction.

The compound of formula III may be isolated and purified by conventional techniques if desired, but it will often be convenient to carry out the reaction with the nitrone of formula IV without isolation of the metal alkyne.

The nitrone of formula IV may be prepared for example by reaction of an aldehyde of formula $R^6CHO$ (VI) with a hydroxylamine of formula $R^7NHOH$ (VII).

The reagents are conveniently stirred together in a solvent. Suitable solvents include polar solvents such as alcohols, eg methanol and ethanol and non-polar solvents such as hydrocarbons, eg toluene.

The reaction conveniently takes place at room temperature, although heating or cooling may be employed to regulate the reaction. In particular the application of heating may facilitate the reaction by permitting water eliminated in the reaction to be removed eg by azeotropic distillation. Thus the reaction

may be carried out at temperatures up to the boiling point of the solvent, eg 0-100 °C. The reaction is usually complete in about 15 min to 2 h. It may be desirable to employ an inert atmosphere, eg of nitrogen or argon.

Other methods for the preparation of nitrones are known in the chemical literature and these may equally be employed if desired.

The nitrone may be isolated and purified by conventional techniques if desired, but it will often be convenient to carry out the reaction with the metal alkyne without isolation of the nitrone.

The reaction between the metal alkyne of formula III and the nitrone of formula IV is generally complete in from 1 to 24 h. Heating or cooling may be employed to regulate the reaction which may take place for example at temperatures from -70 °C up to the boiling point of the solvent, for example from 0-100 °C, but it will generally be convenient to carry out the reaction at room temperature. The reaction is advantageously carried out in an inert atmosphere, for example of nitrogen or argon.

Suitable solvents for the reaction include polar protic or aprotic solvents, conveniently selected from those listed above in respect of the preparation of the metal alkyne.

Thus a convenient way of carrying out the process of the invention involves stirring the aldehyde of formula VI and the hydroxylamine of formula VII in a solvent such as methanol or ethanol for between 15 min and 2 h, conveniently about 30 min to 1 h at room temperature followed by addition of copper (I) chloride, ammonia or diethanolamine and the alkyne of formula V, which latter reagents may be added in any order, followed by stirring for between 1 and 24 h, conveniently at room temperature, the reaction being carried out throughout under an atmosphere of nitrogen or argon, and heating or cooling being employed at any stage if required.

An alternative way of carrying out the reaction involves stirring the aldehyde of formula VI and the hydroxylamine of formula VII in a hydrocarbon solvent, eg toluene, for between 15 min and 1 hr with heating to the boiling point of the solvent (eg to about 112-114 °C in the case of toluene) to remove eliminated water by distillation followed by addition of DMSO or DMF as cosolvent and complexing agent, copper (I) chloride and the alkyne, with stirring for between 15 min and 1 h, the reaction being carried out throughout under an atmosphere of nitrogen or argon. This method is particularly useful for example when any of the reagents contain water sensitive groups, eg acetal groups.

References to room or ambient temperature in the above discussion and elsewhere herein mean temperatures at or about 25 °C, it being understood that the exact temperature will not be critical to satisfactory performance of the reaction.

The compound of formula II may be isolated by conventional means, for example by treatment with dilute acid (e.g. HCl) followed by extraction with an organic solvent e.g. ethyl acetate or chloroform. It may be purified by conventional means e.g. crystallisation or chromatography (e.g. medium or high pressure liquid chromatography).

In the formula I, the group $R^1$ may have various meanings, for example as described in published European Patent Applications 30032, 54917 and 71908. A preferred meaning for $R^1$ in formula I is a (1-4C)-alkyl group optionally substituted by hydroxy groups or halogen, particularly fluorine, atoms. A particularly preferred meaning for $R^1$ is the 1-hydroxyethyl group.

In the formulae II, III and V, $R^2$ may correspond to the group $R^1$ required in the final compound of formula I or may be a protected form of such a group. For example where $R^1$ contains a hydroxy group (e.g. where $R^1$ is a 1-hydroxyethyl group) the hydroxy group may be protected by a hydroxyl protecting group. $R^2$ may alternatively represent a precursor for the group $R^1$ as hereinbefore discussed, in the sense of a group readily convertible thereinto by one or more reactions, for example an acyl group or a protected form thereof, or an alkenyl group. For example a precursor of the 1-hydroxyethyl group may be the acetyl group or a protected form thereof, e.g. the acetal, or an ethenyl group ($CH_2=CH$-). Such groups may be transformed into the 1-hydroxyethyl group by known methods.

An example of a cyclisation reaction leading to the formation of a compound of formula I is given in Scheme A, derived from published European Patent Application 126587 (EP 126587), in which the groups $R^1$, $R^2$, $R^3$, $R^4$ and $R^5$ are as herein defined and $R^{10}$ is a carboxyl protecting group.
Each of the stages (a) to (f) of scheme A may be carried out, for example, by the methods described in EP 126587.

The process of the invention may produce precursors of the compounds of formula A of scheme A wherein the nitrogen atom in the ring is protected by an amino protecting group and/or in which the carboxyl group is protected. The process of the invention may also produce compounds readily convertible by known methods into the said precursors of the compounds of formula A, for example compounds corresponding to the formula A but wherein the carboxy group is replaced by a hydroxymethyl group (wherein the hydroxy group may be protected) or a formyl group (wherein the oxo group is protected, e.g.

as an acetal thereof), or compounds wherein the group $C(R^3)(R^4)COOH$ in formula A is replaced by a group -COOH or a protected form thereof and the nitrogen atom in the ring is protected by an amino protecting group. Such precursor compounds may readily be converted by known methods (e.g. as described in EP 126587, Heterocycles 21 29 (1984) and Tetrahedron Letters 26 4739 (1985) and 21 2783 (1980)) into the compounds of formula A.

The process of the invention may also produce precursors of the compounds of formula B of scheme A wherein the nitrogen atom in the ring is protected by an amino protecting group and/or the carboxyl group is protected by a carboxyl protecting group and/or the oxo group is protected, e.g. as an acetal thereof.

Further examples of methods useful for cyclisation of azetidinones to form carbapenem compounds are given in published European patent application no. 188 816, and the process of the invention is applicable also for the production of azetidinones useful as starting materials in the processes set out therein, and precursors therefor. For example compounds wherein $R^6$ is of the formula $-C(R^3)(R^4)COOH$ and $R^7$ is of the formula $-CH_2COOH$ or wherein $R^6$ and $R^7$ represent protected forms of the said groups.

Published European patent applications nos. 180 189 and 192 171 describe methods for selective reduction of certain bicyclic beta-lactam ring structures containing an exocyclic double bond alpha to the beta-lactam ring. The process of the invention may also be employed to prepare precursors of such compounds. An example of such a precursor compound is a compound wherein $R^6$ is of the formula $-C(R^3)(ZR^a)COOH$ wherein Z may be oxygen, sulphur or selenium, $R^a$ is (1-4C)alkyl eg methyl, or phenyl, wherein the COOH moiety may be protected, or such a group wherein the COOH moiety is replaced by hydroxymethyl (wherein the hydroxy group may be protected) or -CHO (wherein the oxo group is protected, eg as an acetal thereof).

The groups $R^3$ and $R^4$ (which may be the same or different) may be chosen from any of the wide variety of such groups mentioned in the literature, for example in published European Patent Applications 30032, 54917 and 71908. Preferred meanings for $R^3$ and $R^4$ are hydrogen or (1-4C)alkyl groups, particularly-methyl groups. Especially preferred as antibiotics are compounds of the formula I wherein $R^3$ and $R^4$ are both hydrogen or wherein $R^3$ is methyl and $R^4$ is hydrogen.

The preferred compounds of formula I are those wherein, of the groups $R^1$, $R^3$ and $R^4$, $R^1$ represents the group $CH_3CHOH$, $R^3$ represents H or $CH_3$ and $R^4$ represents H. Such compounds advantageously have the sterochemistry denoted by one of the formulae Ia or Ib (wherein $R^5$ is as hereinbefore defined). Accordingly the process of the invention is advantageously operated in such a way that compounds of the formula IIa (wherein, when a compound of formula Ia is required, $R^6$ has the desired stereochemistry in respect of the methyl group $R^3$) are produced, if necessary after subsequent treatment and/or separation of isomers.

In the Examples given hereinafter, the stereochemistry of the reagents and products is indicated where appropriate. In the case of centres marked with the * symbol the relative stereochemistry only is implied.

$R^5$ may be one of a variety of groups known from the literature, for example published European Patent Applications 30032, 54917 and 71908. Particularly preferred are the compounds wherein $R^5$ represents the group $-S(CH_2)_2NH_2$ and tautomers thereof ("thienamycin") or $-S(CH_2)_2NHCH=NH$ and tautomers thereof ("imipenem"), in the said compounds $R^1$ representing $CH_3CHOH$ and $R^3$ and $R^4$ representing hydrogen; and the compound wherein $R^5$ represents a group $-SCH_2C(:NH)(N(CH_3)_2)$ and tautomers thereof wherein $R^1$ represents $CH_3CHOH$, $R^3$ represents methyl and $R^4$ represents hydrogen. Also preferred are the compounds described in EP 126587 which are of the formula VIII wherein $R^{30}$ represents a hydrogen atom, a 1-hydroxyethyl group or a 1-hydroxyethyl group in which the hydroxy group is protected with a protecting group; $R^{31}$ represents a hydrogen atom or an alkyl group having 1 to 3 carbon atoms; $R^{32}$ represents a hydrogen atom or a protecting group for an amino group; $R^{33}$ represents a hydrogen atom or a protecting group for a carboxyl group and Y represents a group of the formula $-N(R^{35})(R^{36})$ wherein $R^{35}$ and $R^{36}$ which may be the same or different, each represents a hydrogen atom, an alkyl group having 1 to 5 carbon atoms, an alkenyl group having 3 to 4 carbon atoms, an aralkyl group having 1 to 3 carbon atoms in its alkyl moiety, a substituted alkyl group having 1 to 5 carbon atoms or a pyridyl group, or $R^{35}$ and $R^{36}$ taken together represent an alkylene chain or an alkylene chain containing an oxygen atom, a sulphur atom or a $(C_1-C_3)$alkyl-substituted nitrogen atom to form, together with the adjacent nitrogen atom, a substituted or unsubstituted 3- to 7-membered cyclic amino group which may contain double bond(s) in its ring, a substituted or unsubstituted guanidyl group of the formula IX wherein R37 represents a hydrogen atom or an alkyl group having 1 to 3 carbon atoms, a protected or unprotected hydroxyl group, an alkoxy group having 1 to 3 carbon atoms, an unsubstituted or $(C_1-C_3)$alkyl-substituted hydrazino group or a group of the formula $-NHOR^{38}$ wherein $R^{38}$ represents a hydrogen atom, a protecting group for a hydroxyl group or an alkyl group having 1 to 3 carbon atoms, and pharmacologically acceptable salts thereof.

It will be understood that as the identity of the group $R^5$ plays no part in the process of the invention,

the process is equally applicable to the production of intermediates for use in the manufacture of any compound of formula I, whatever the identity of the group $R^5$.

References herein to protecting groups and protection of functional groups mean that the functional group in question carries a group readily removable to provide the desired functional group. Examples of protecting groups and their methods of removal appear in the literature.

When reference is made to protecting groups being present at any position in the compounds described herein such protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower" signifies that the group to which it is applied preferably has 1-4 carbon atoms.

Examples of carboxyl protecting groups include straight or branched chain (1-12C)alkyl groups (eg methyl, ethyl isopropyl, t-butyl); halo lower alkyl groups (eg 2-iodoethyl, 2,2,2-trichloroethyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxycarbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); and (2-6C)alkenyl groups (eg vinylethyl).

Methods particularly appropriate for the removal of carboxyl protecting groups include for example acid-, base- or enzymatically-catalysed hydrolysis.

Examples of hydroxyl protecting groups include lower alkoxycarbonyl groups (eg t-butoxycarbonyl); halo lower alkoxycarbonyl groups (eg 2-iodoethoxycarbonyl, 2,2,2-trichloroethoxycarbonyl); aryl lower alkoxycarbonyl groups (eg benzoyloxycarbonyl, p-methoxybenzyloxycarbonyl, o-nitrobenzyloxycarbonyl, p-nitrobenzyloxycarbonyl); tri lower alkylsilyl (eg trimethylsilyl, t-butyldimethylsilyl) and aryl lower alkyl (eg benzyl) groups.

Oxo groups are conveniently protected in the form of acetals thereof, which may be acyclic or cyclic.

Examples of amino protecting groups include aralkyl groups (eg benzyl and substituted benzyl, eg p-methoxybenzyl and 2,4-dimethoxybenzyl, and triphenylmethyl); di-p-anisylmethyl and furylmethyl groups; acyl (eg alkoxycarbonyl and aralkoxycarbonyl eg t-butoxycarbonyl and benzyloxycarbonyl); trialkylsilyl (eg trimethylsilyl and t-butyldimethylsilyl); alkylidene (eg methylidene); benzylidene and substituted benzylidene groups; and the phthalimido group.

The following Examples are provided to illustrate the invention and its utility, but do not imply any limitation of the scope thereof.

Nmr means nuclear (proton) magnetic resonance spectroscopy, spectra being quoted in delta in parts per million relative to tetramethylsilane (delta = 0) as internal standard (s = singlet, d = doublet, t = triplet, q = quartet, m = multiplet, br = broad). Spectra are measured at a field strength of $200MH_z$ unless otherwise stated. J = coupling constant in Hz.

Mass spectral data were obtained by fast atom bombardment (FAB), chemical ionization (CI) or electron impact (EI). Mass values are quoted in Daltons.

Mplc and hplc mean medium and high pressure liquid chromatography respectively. Tlc means thin layer chromatography.

The following Examples illustrate the process of the invention.

Example 1

1-Benzyl-4-(2-benzyloxyethyl)-3-(1-hydroxyethyl)azetidin-2-one

To a solution of N-benzylhydroxylamine (3.1g) in ethanol (80ml), under argon, was added 3-benzyloxypropanal (4.45g). The clear solution was stirred for 30 min at ambient temperature and then a solution of butyn-3-ol in water (c 45%, 5ml) was added, followed by copper (I) chloride (3.5g) and concentrated aqueous ammonia (20ml). After stirring at ambient temperature for 2 hrs the mixture was added to hydrochloric acid (2M, 200ml), and then extracted with ethyl acetate (2 x 300ml). The organic extracts were combined and extracted with hydrochloric acid (2M, 100ml) and then saturated aqueous sodium chloride (2 x 100ml). The organic layer was dried over magnesium sulphate and evaporated to dryness. The residue

was purified by mplc cn silica using a gradient of ethyl acetate/hexane 1:4 changing linearly to ethyl acetate 100%. The appropriate fractions were evaporated to dryness to give 3.7g (44%) of a mixture of cis and trans isomers of the title compound as a clear oil.

A sample of the cis isomer isolated as above had the following nmr in $CDCl_3$ at 400 MHz:
1.45 (d, 3H); 1.8 (m, 1H); 2.1 (m, 1H); 3.2 (q, J = 5.5 and 11.0, 1H); 3.3 (m, 1H); 3.55 (m, 1H); 3.6 (m, 1H); 4.1 (m + d, 2H); 4.45, (s, 2H); 4.55 (d, 1H); 7.25 (m, 10H); M + H (FAB) = 340.

Example 2
1-Benzyl-4-(2-benzyloxy-1-methylethyl)-3-(1-hydroxyethyl)azetidin-2-one

3-(Benzyloxy)-2-methylpropanal (3.60g) and N-benzylhydroxylamine (2.50g) were stirred together in 50ml ethanol under argon at ambient temperature for 45mins. To the resulting clear solution were added sequentially, while still stirring under argon, 5ml of a ca. 45% solution of butyn-3-ol, 3.0g of copper (I) chloride and 16ml concentrated aqueous ammonia.

The pale green clear reaction mixture was stirred for 3hrs at ambient temperature under argon and then quenched in 200ml 2N hydrochloric acid, extracted with 2 x 300ml ethyl acetate and the organic extracts combined and washed with 2N hydrochloric acid (50ml), water (200ml) and saturated aqueous brine (100ml). Filtration through anhydrous sodium sulphate and evaporation in vacuo left 8.43g of a yellow oil.

The residual oil was purified by mplc on silica using a 1:1 mixture of hexane/ethyl acetate to give 5.16g of the title compound as a near colourless oil (73%). Tlc on silica using hexane/ethyl acetate (1:1) as eluant showed two spots in approximately equal ratio of Rf's 0.12 and 0.15, which are the $C_{3,4}$ trans and cis isomers respectively.

A sample of the cis isomer isolated as above had the following nmr in $CDCl_3$:
0.98 (d, 3H); 1.45 (d, 3H); 1.70 (broad s, 1H); 2.45 (m, 1H); 3.18 (q, J = 5.5 and 10.5, 2H); 3.40 (q, 1H); 3.46 (q, 1H); 4.03 (d, 1H); 4.17 (sextet, 1H); 4.46 (s, 2M); 4.82 (d, 1H); 7.38 (m, 10H); M + H (CI) = 354.

Example 3
3-Acetyl-1-benzyl-4-(2-benzyloxy-1-methylethyl)trans-azetidin-2-one

3-(Benzyloxy)-2-methylpropanal (1.80g) and N-benzylhydroxylamine (1.35g) were stirred together in 40ml ethanol under argon at ambient temperature for 1hr. To the resulting clear soluton was added 1.40g copper (I) chloride and 4ml 50% aqueous diethanolamine. After 5mins (under argon), 0.80g (0.92ml) butyn-2-one was added dropwise in 5ml ethanol over ca. 20 mins, stirring under argon. After a further 2hrs the reaction mixture was poured into 100ml 2N hydrochloric acid and extracted with ethyl acetate (2 x 150ml). The organic extracts were washed sequentially with 2N hydrochloric acid, water and saturated brine and filtered through anhydrous sodium sulphate. Evaporation in vacuo gave 2.95g of a yellow oil. Purification by mplc using ethyl acetate/ hexane gave 2.07g of the title compound as a near colourless oil (58%). The material was single spot by tlc., silica;ethyl acetate/hexane 1:1, Rf 0.50. N.m.r. in $CDCl_3$ 0.90 (t, 3H); 2.04 (m, 1H); 2.20 (d, 3H, cis/trans isomers at $C_{4,5}$); 3.23 (q, 1H); 3.33 (m, 1H); 3.89 (q, J = 2.5 and 6.5, part 1H, 1 isomer); 4.05 (m, 1.5H); 4.15 (d, part 1H, 1 isomer); 4.18 (m, 1H); 4.37 (unresolved d, 1H); 4.64 (q, 1H); 7.25 (m, 10H); M + H (CI) = 352.

Example 4
1-Benzyl-3-acetyl-4-[(3-methoxycarbonyl)-2,2-(ethylenedioxy)propyl]-trans-azetidin-2-one

A mixture of Methyl 3,3-(ethylenedioxy)-4-formylbutyrate (2.1g) and benzylhydroxylamine (1.2g) was heated at 80°C in toluene (30ml) for 30 min and then evaporated under reduced pressure. The residue was dissolved in toluene (30ml) and dimethylsulphoxide (8ml) added. To the stirred mixture under argon was added copper (I) chloride (1.2g) and then butyn-3-one (0.4g) was added dropwise over 10 min. The mixture was allowed to stir for 20 min, and then saturated aqueous ammonium chloride solution (30ml) was added. Concentrated aqueous ammonium hydroxide was also added until all solid material had dissolved and then the solution was extracted with diethyl ether (2 x 35ml). The organic extract was washed with saturated sodium chloride solutions dried with magnesium sulphate and evaported to dryness. The residue was purified by medium pressure liquid chromatography on silica gel using a gradient of ethyl acetate/hexane (1:4) rising to (3:1). The appropriate fractions evaporated to dryness gave the title compound (0.5g) as a yellow oil.

A sample isolated by the above chromatography had the following nmr in $CDCl_3$:
2.3 (s,3H); 2.55 (s,2H); 3.65(s,3H); 3.65-4.15 (c,8H); 4.2 (d,1H); 4.55 (d,1H); 7.3 (c,5H). M+H(CI) = 340.

The product of Example 4 may be N-deprotected and cyclised to yield a carbapenem of formula (I) by known methods for example as described in J. Org. Chem. 47, 2328 (1982); Chem. Commun, 104 (1982).

Example 5
1-Benzyl-4-(2,2-diethoxyethyl)-3-(1-hydroxyethyl)azetedin-2-one.

To a stirred solution of N-benzylhydroxylamine (1.0g) in ethanol (30ml) under argon was added 3,3-diethoxypropionaldehyde (1.0g). The solution was stirred for 1h at ambient temperature and then a solution of butyn-3-ol in water (45%, 2.0ml) was added, followed by copper (I) chloride (1.0g) and concentrated aqueous ammonia (8ml). After stirring at ambient temperature for 18 h the mixture was added to saturated aqueous ammonium chloride solution (50ml) and then extracted with ethyl acetate (2 x 50ml). The organic extracts were combined and extracted with saturated aqueous ammonium chloride (50ml) and then saturated aqueous sodium chloride (2 x 50ml). The organic layer was dried over magnesium sulphate and evaporated to dryness. The residue was purified by chromatography on silica using ethyl acetate/hexane 1:1. The appropriate fractions were evaporated to dryness to give 500mg (22%) of the title compound.

A sample had the following Nmr in deuterochloroform:
1.15 (m,6H); 1.3 (d,3H); 1.72 (m,1H); 2.02 (m,1H); 2.74 (s,1H); 2.95 (m,1H); 3.50 (m,5H); 4.08 (m,1H); 4.15 (d,1H); 4.61 (d,1H); 4.51 (m,1H); 7.28 (m,5H).
M + H(CI) = 322.

Example 6
3-Acetyl-1-benzyl-4-(2,2-diethoxyethyl)-trans azetedin-2-one.

To a solution of N-benzyl hydroxylamine (2.30g) in ethanol (70ml) under argon was added 3,3-diethoxy propionaldehyde. The solution was stirred for 1h at ambient temperature and then a 50% aqueous solution of diethanolamine (5.0ml) was added followed by copper (I) chloride. The mixture was cooled in an icebath then butyn-3-one (1.70g) was added dropwise. After stirring at ambient temperature for 18 h the mixture was added to saturated aqueous ammonium chloride (100ml) and then extracted with ethyl acetate (3 x 100ml). The organic extracts were combined and extracted with saturated aqueous ammonium chloride (100ml) and then saturated aqueous sodium chloride (2 x 100ml). The organic layer was dried over magnesium sulphate and evaporated to dryness. The residue was purified by chromatography on silica using a gradient of ethyl acetate/hexane 1:3 changing linearly to ethyl acetate/hexane 3:1. The appropriate fractions were evaporated to dryness to give 1.15g (24%) of the title compound as a yellow oil.

A sample had the following Nmr in deuterochloroform:
1.1 (m,6H); 1.72 (m,1H); 1.97 (m,1H); 2.28 (s,3H); 3.45 (m,4H); 4.00 (m,1H); 4.05 (d,1H); 4.20 (d,1H); 4.58 (d,1H); 4.40 (m,1H); 7.30 (m,5H).
M + H(CI) = 320.

Example 7
3-Acetyl-1-benzyl-4-(2-methoxycarbonylethyl)azetidin-2-one.

To a stirred solution of N-benzyl hyroxylamine (3.0g) in ethanol (100ml) under argon was added 3-methoxycarbonyl propionaldehyde (2.32g). The solution was stirred for 1h at ambient temperature, and then a 50% aqueous solution of diethanolamine (5ml) was added followed by copper (I) chloride. The mixture was cooled in an icebath then butyn-3-one (2.15g) was added dropwise. After stirring at ambient temperature for 18h the mixture was added to saturated ammonium chloride solution (150ml) containing concentrated ammonia (10ml, S.G. 0.880) and then extracted with diethyl ether (3 x 100ml). The organic extracts were combined and extracted with saturated ammonium chloride solution (100ml), then saturated sodium chloride solution (2 x 100ml). The organic layer was dried over magnesium sulphate and evaporated to dryness. The residue was purified by chromatography on silica using a gradient of ethyl acetate/hexane 1:4 changing linearly to ethyl acetate/hexane 2:1. The appropriate fractions were evaporated to dryness to give 1.50g (26%) of the title compound as an orange oil.

The sample had the following Nmr in deuterochloroform:
1.75 (m,2H); 2.24 (m,2H); 2.3 (s,3H); 3.65 (s,3H); 3.89 (m,2H); 4.18 (d,1H); 4.6 (d,1H); 7.3 (m,5H).
M + H(EI) = 290.

Example 8
1-p-methoxybenzyl-3-acetyl-4-(2,2-diethoxy-1R *-methylthio-1R *-methylethyl)-trans azetedin-2-one.

N-4-methoxybenzylhydroxylamine, (7.90g, 0.05 mole) and 3,3-diethoxy-2-methyl-2-methylthio propionaldehyde (12.0g, 0.058 mole) were dissolved in 80ml toluene (pre-dried over 4A molecular sieves) in a 250ml 3-neck flask and the flask immersed in an oil bath pre-heated to 150° C. The mixture was refluxed for 30mins under a Dean and Stark trap removing 10ml distillate after 5min and 10ml further after 20min rection. The flask was then removed from the oil bath and cooled in an icebath under argon.

To the resulting pale straw coloured solution of nitrone was added successively 10ml DMSO, and 5.5g copper (I) chloride followed by 95% butynone at 5-15° C (max)(4.0ml, 50 mmole) in 0.2ml portions over 30 mins. After stirring at room temperature for 2h the reaction mixture was poured into a mixture of concentrated ammonia solution (S.G. 0.880) 25ml saturated $NH_4Cl$ solution, 300ml water and 200ml ether and stirred for 10 mins.

The organic layer was separated and the aqueous layer re-extracted with ether (2 x 100ml). The organics were washed successively with water, dilute $NH_4OH$, 0.1NHCl (2X), water and brine. The residue was filtered through anhydrous sodium sulphate and evaporated in vacuo to give 15.84g of a brown oil. Back extraction of the acid washes yielded a further 840mg of the product.

Purification by medium pressure liquid chromatography on silica gel using ethyl acetate/hexane 1:4 rising to 1:1 gave 9.71g, of the title compound as a pale yellow oil (47%). A further 1.26g material was obtained from later fractions of a 80-90% purity. A sample isolated by the above procedure had the following Nmr in $CDCl_3$: 1.09/1.22 (t,3H/t,3H); 1.19 (s,3H); 1.94 (s,3H) (satellite at 2.04, s, 1/11.5,3H); 2.28 (s,3H); 3.27 (sextet,1H); 3.18 (sextet,3H); 3.80 (s,3H); 4.22 (d,J=2.5,1H); 4.30 (s,1H); 4.33 (d,J=4.5,1H); 4.43 (d,J=2.5,1H); 4.81 (d,J=4.5,1H); 6.87 (d,2H); 7.20 (d,2H)ppm. This spectrum showed the product to be a mixture of IR * and IS * isomers at the C-3,1' position in the ratio of 11.5:1. The material was single spot on t.l.c., silica; ethyl acetate/hexane 1:1, Rf 0.50.

M+H (CI)=410.

3,3-Diethoxy-2-methyl-2-methylthiopropionaldehyde, used as starting material in Example 8, may be prepared as follows:

Dimethyldisulphide 23.6g (0.25mole) was stirred in 100ml $CH_2Cl_2$ at -20° C under argon. Sulphuryl chloride (36g, 0.26mole) was added dropwise for 10 min at -15° max and then at -15° C for 2h. To the deep orange solution of methyl sulphenyl chloride so obtained was added 57.0 (0.50mole) 3-ethoxymethacrolein in 50ml $CH_2Cl_2$, at -15° to -20° C over 15 min.

The reaction mixture was stirred at -15° C for a further 1h and at 0° C for 1h. While stirring well at 0° C a solution of 1.6 moles of Na in 200ml ethanol was added over 20 min, and the thick white reaction mixture stirred to room temperature for 1h.

After filtration, 30g solid sodium bicarbonate was added and the mixture evaporated in vacuo at 30° C max. The residue was partitioned between 500ml water and 200ml ether and the organic layer washed with water (2X) and saturated brine. Filtration through anhydrous sodium sulphate and evaporation in vacuo gave 98.8g of a yellow oil. Distillation of the crude product in vacuo gave 21g recovered starting material and impurities plus 649g 3,3-diethoxy-2-methyl-2-methylthiopropionaldehyde as a near colourless oil (63%). The sample showed the following nmr in $CDCl_3$: 1.20-1.40 (br,m,9H); 1.88 (s,3H); 3.72 (m,4H); 4.60 (s,1H); 9.26 (s,1H)ppm.

M+H (CI)=207 and M+$NH_4$-$H_2O$=206.

Example 9

1-t-Butoxycarbonylmethyl-3-acetyl-4-[(1R *-methylthio-1R *-methyl-2,2-diethoxy)ethyl]-trans-azetidin-2-one.

To N-hydroxy-t-butylglycine (10g) in toluene (100ml) was added 1-thiomethyl-1-methyl-2,2-diethoxypropanal (15.2g). The mixture was refluxed under a water separator until evolution of water ceased. To the cooled mixture was added dimethylsulphoxide (30ml) and the mixture cooled to about 5° C. Copper (I) chloride (7.95g) was added. To the mixture under argon was added butyn-3-one dropwise. After the addition the mixture was stirred for 1h at room temperature then saturated aqueous ammonium chloride solution (150ml) was added. Concentrated aqueous ammonium hydroxide was also added until all solid material had dissolved and then the solution was extracted with diethyl ether (150ml). The organic extract was washed with saturated sodium chloride solution, dried with magnesium sulphate and evaporated to dryness. The residue was purified by chromotagraphy on silica gel using a gradient of hexane to 30% ethyl acetate/hexane (v/v) to give 10.2g of the title compound as an oil.

A sample isolated by the above chromatography had the following Nmr in Deuterochloroform. 1.2 (m,6H); 1.45 (s,9H); 2.15 (s,3H); 2.23 (s,3H); 3.4-3.8 (m,4H); 3.97 (d,1H); 4.2 (d,1H); 4.28 (d,1H); 4.35 (s,1H); 4.55 (d,1H).

Example 10

1-t-Butoxycarbonylmethyl-3-acetyl-4-[(1R *-methylsulphinyl-1R *(methyl-2,2-diethoxy)ethyl]-trans-azetidin-2-one.

To 1-t-butoxycarbonylmethyl-3-acetyl-4-[1-methylthio-1-methyl-2,2-diethoxy)ethyl]-trans-azetidin-2-one (0.8g) in ethanol (10ml) was added sodium periodate (0.47g) in water (8ml) at 0°C. The resulting mixture was stirred for 18h. at room temperature, water (20ml) added and the mixture extracted with dichloromethane (25ml). The organic extract was evaporated to dryness and purified by chromatography on silica gel using a gradient of ethyl acetate changing to 20% ethanol/ethyl acetate (v/v). The appropriate fractions were evaporated to dryness to give 0.7g of the title compound.

A sample isolated by the above chromatography had the following Nmr in Deuterochloroform. 1.25 (m,9H); 1.48 (s,9H); 2.31 (s,3H); 2.45 (s,3H); 3.4-3.8 (m,4H); 3.85 (d,1H); 4.32 (d,1H); 4.38 (s,1H); 4.65 (d,1H); 4.92 (d,1H).

M + H(EI) = 419.

The product of the above reaction(0.5g) was heated in xylene (10ml) at about 130°C for 1hr., evaporated to dryness and purified by chromatography on silica gel using a gradient of 10% ethyl acetate/hexane changing to ethyl acetate. The appropriate fractions were evaporated to dryness to give 1-t-butoxycarbonylmethyl-3-acetyl-4-[1-(1,1-diethoxymethyl)ethenyl] trans-azetidin-2-one (0.15g) and 1-t-butoxycarbonylmethyl-3-acetyl-4-(1-formylethenyl)trans-azetidin-2-one (0.075g).

A sample of acetal isolated by the above chromatography had the following Nmr in Deuterochloroform. 1.2 (m,6H); 1.4 (s,9H); 2.32 (s,3H); 3.4-3.7 (m,4H); 3.5 (d,1H); 4.08 (d,1H); 4.2 (d,1H); 4.65 (d,1H); 4.7 (s,1H); 5.2 (s,1H); 5.4 (s,1H).

A sample of aldehyde isolated by the above chromatography had the following resonances Nmr in Deuterochloroform. 1.4 (s,9H); 2.36 (s,3H); 3.5 (d,1H); 4.12 (d,1H); 4.18 (d,1H); 5.0 (d,1H); 6.3 (s,1H); 6.52 (s,1H); 9.65 (s,1H).

Treatment of the acetal obtained above, with mild acid, leads to complete conversion to the corresponding aldehyde described.

The olefinic products described above, may be catalytically reduced and hence cyclised to carbapenems of formula (I) bearing the optional 1-(β)-methyl substituent (of formula Ia) by known methods for example as described in EP 0180189.

The following Examples illustrate the means by which compounds of formula II prepared by the process of the invention may be converted into the carbapenems of formula I.

Example A

(a) 3-Acetyl-1-benzyl-4-(2-benzyloxyethyl)trans-azetidin-2-one

To 1-benzyl-4-(2-benzyloxyethyl)-3-(1-hydroxyethyl)azetidin-2-one (2.7g) (prepared by the method of Example 1) in acetone (30ml) at ambient temperature was added dropwise 5ml of chromic acid (prepared from chromium trioxide (2.7g) in sulphuric acid (2.3ml), diluted to 10ml with water). Sufficient water was added to dissolve the resultant chromium salts, the mixture was saturated with sodium chloride, and extracted with ethyl acetate (30ml). The organic extracts were washed with saturated aqueous sodium chloride, dried over magnesium sulphate and evaporated to dryness. The residue was purified by mplc on silica using a gradient of 10% ethyl acetate/hexane increasing to 60% ethyl acetate/hexane. The appropriate fractions evaporated to dryness gave 1.8g (67%) of the title compound as a clear oil. Nmr in $CDCl_3$ 1.7 (m, 1H); 1.9 (m, 1H); 2.2 (s, 3H); 3.4 (m, 2H); 4.0 (m, 2H); 4.2 (d, 1H); 4.35 (s, 2H); 4.55 (d, 1H); 7.25 (m, 10H); M + H = 337 EI.

(b) 1-Benzyl-4-(2R *-benzyloxyethyl)-3S *-(1R *-hydroxyethyl)-trans-azetidin-2-one

To the product of (a) above (1.7g) in diethyl ether (100ml) under argon, was added powdered potassium iodide (1.2g). The mixture was stirred for 1hr. at ambient temperature, and then a solution of potassium tri-sec-butylborohydride (1molar) in tetrahydrofuran (13.5ml) was added dropwise. The resulting mixture was stirred for 2hrs. 2M Hydrochloric acid (50ml) was added slowly, the organic layer removed and the aqueous layer extracted with diethyl ether (100ml). The organic layers were combined and washed with saturated aqueous sodium chloride, dried over magnesium sulphate and evaporated to dryness. The residue was purified by mplc on silica using a gradient of 30% ethyl acetate/hexane increasing to 100% ethyl acetate. The appropriate fractions evaporated to dryness gave the title compound as a clear oil (1.5g, 88%), nmr in $CDCl_3$ 1.28 (d, 3H); 1.7 (m, 1H); 1.95 (m, 1H); 2.93 (q, J = 2.0 and 6.5, 1H); 3.5 (t, 2H); 3.6 (dq, 1H); 4.05 (m, 1H); 4.15 (d, 1H), 4.4 (s, 2H); 4.6 (d, 1H), 7.25 (m, 10H); M + H (FAB) = 340.

(c) 1-Benzyl-4R *-(2-benzyloxyethyl)-3S *-(1R *-t-butyldimethylsilyloxyethyl)trans-azetidin -2-one

To the product of (b) above (1.1g) in dimethylformamide (5ml) at ambient temperature was added imidazole (0.65g) and t-butylchlorodimethylsilane (0.96g). The mixture was stirred under argon for 4 hrs. Water (20ml) was added, and the mixture extracted with diethyl ether (2 x 20ml). The organic extracts were dried with magnesium sulphate and evaporated to dryness. The residue was purified by mplc on silica using a gradient of 10% ethyl acetate/hexane increasing to 25% ethyl acetate/hexane. The appropriate fractions evaporated to dryness gave the title compound as a clear oil (1.05g, 72%), nmr in $CDCl_3$ 0.05 (s, 3H); 0.08 (s, 3H); 0.9 (s, 9H); 1.2 (d, 3H); 1.8 (m, 1H); 2.0 (m. 1H) 2.87 (q, J = 2.0 and 5.0, 1H); 3.42 (t, 2H); 3.73 (hep, 1H); 4.2 (m, 1H); 4.25 (d, 1H); 4.4 (s, 2H); 4.5 (d, 1H); 7.7 (m, 10H), M + H (FAB) = 454.

(d) 3S *-(1R *-t-butyldimethylsilyloxyethyl)-4R *-(2-hydroxy ethyl)trans-azetidin-2-one

To the product of (c) above (2.5g) in a mixture of diethyl ether (5ml) and liquid ammonia (40ml) was added, in small portions, sodium (0.5g) until a blue colouration persisted. Ammonium chloride (1.5g) was added in portions, and the ammonia allowed to evaporate. To the residue was added water (10ml) and the resulting mixture was extracted with chloroform (2 x 25ml). The organic extracts were dried with magnesium sulphate and evaporated to dryness. The residue was purified by mplc on silica using a gradient of 25% ethyl acetate/hexane increasing to 70% ethyl acetate/hexane. The appropriate fractions evaporated to dryness gave the title compound (0.75g), 50% as a clear oil, nmr in $CDCl_3$ 0.1 (d, 3H), 0.85 (s, 9H), 1.38 (d, 3H), 1.85 (q, 2H), 2.9 (dq, 1H); 3.65 (hex, 1H); 3.75 (m, 2H); 4.15 (m, 1H); 6.1 (br.s, 1H); M + H (CI) = 274.

(e) 3S *-(1R *-t-butyldimethylsilyloxyethyl)-4S *-(1-carboxy-methyl)trans-azetidin-2-one

To the product of (d) above (0.6g) in pyridine (6ml) was added a solution of chromium trioxide in pyridine (from chromium trioxide (1.3g) in water (1ml) added to pyridine (12ml) at ice-bath temperature). The resulting mixture was stirred at ambient temperature for 24 hrs. Water (20ml) was then added, and the mixture treated with sufficient aqueous sodium hydrogen sulphite to remove excess chromate. The mixture was then evaporated to low volume and the pH adjusted to 3 with dilute hydrochloric acid. The mixture was extracted with ethyl acetate (3 x 30ml), the extracts washed with saturated aqueous sodium chloride, dried and evaporated to dryness. The residue was dissolved in dichloromethane (30ml) and extracted with aqueous potassium carbonate (15ml). The aqueous layer was washed with dichloromethane, acidified to pH 3 with dilute hydrochloric acid, and extracted with dichloromethane (2 x 10ml). The organic extracts were dried over magnesium sulphate and evaporated to dryness to give the title compound as a white solid (0.04g, 7%), nmr in $DMSOd_6$ 0.04 (s, 3H); 0.05 (s, 3H); 0.85 (s, 9H); 1.13 (d, 3H); 2.80 (q, 1H); 3.75 (m, 1H); 4.1 (m, 1H);

The product of (e) may be cyclised to yield a carbapenem of formula I by known methods e.g. as described in Heterocycles 21, 29 (1984).

## Example B

(a) 3-Acetyl-1-benzyl-4-(2-benzyloxy-1-methylethyl)trans-azetidin-2-one

1-Benzyl-4-(2-benzyloxy-1-methylethyl)-3-(1-hydroxyethyl)azetidin-2-one (7.10g) prepared as in Example 2 (cis/trans isomer mixture) was stirred in 60ml acetone cooled in an ice-bath. 12ml of a solution of 2.67g chromium trioxide (in 2.3ml conc. sulphuric acid and 10ml water) was added dropwise at such a rate as to maintain a reaction temperature of 15-20 °C. After stirring for 1hr the mixture was diluted with 200ml water and extracted with ethyl acetate (2 x 200ml). The organic extracts were washed with water, followed by saturated brine and then filtered through anhydrous sodium sulphate and evaporated in vacuo to give 5.04g of a yellow oil. The residue was purified by mplc on silica using ethyl acetate/hexane (1:1) to give 4.05g of a pale yellow oil, nmr spectrum identical to the compound prepared in Example 3.

(b) 1-Benzyl-4R *-(2-benzyloxy-1RS-methylethyl)-3S *-(1R *-hydroxyethyl)-trans-azetidin-2-one

3-Acetyl-1-benzyl-4-(2-benzyloxy-1-methylethyl)-trans-azetidin-2-one (prepared either by the procedure of Example 3 or that of (a) above) (3.85g) was stirred in 200ml diethyl ether under argon with powdered potassium iodide (2.50g) for 1hr. A solution of potassium tri-sec-butyl borohydride (1M) in tetrahydrofuran (25ml) was added via syringe over about 5mins. The resulting mixture was stirred for 1.5hrs and 50ml 2N hydrochloric acid added cautiously followed by 100ml diethyl ether. The organic layer was separated and washed with water (100ml) and saturated aqueous brine. Filtration through anhydrous sodium sulphate and evaporation in vacuo left 9.30g of a yellow oil.

Purification by mplc on silica using ethyl acetate/hexane (1:1) gave 2.94g of a near colourless oil (76%). Hplc analysis revealed 92% of the 1R *-hydroxyethyl stereoisomer required and 7.2% of the 1S * isomer, nmr in $CDCl_3$ 0.92 (q, 3H, 10% satellites); 1.28 (q, 3H); 2.06 (m, 1H); 2.34 (br s, 1H); 2.98/3.04 (q/q, J = 3.0 and 6.5 each, 1H); 3.35 (m, 2H); 3.50 (q, J = 6.0 and 3.0, part 1H); 3.64 (q, J = 4.0 and

3.0, part 1H); 4.03 (m, 2H); 4.42 (s, 2H, 10% satellite); 4.71 (t, 1H); 7.28 (m, 10H).

(c) 1-Benzyl-4R *-(2-benzyloxy-1RS-methylethyl)-3S *-(1R *-t-butyldimethylsilyloxyethyl)-trans-azetidin-2-one

The product of (b) above (2.13g) was stirred in 20ml sieve dried dimethylformamide together with 1.20g t-butyldimethylsilyl chloride and 1.10g imidazole for 48hrs at ambient temperature. The reaction mixture was partitioned between 300ml water and 200ml ether. The organic phase was washed with water (2 x 300ml) then saturated aqueous brine, filtered through anhydrous sodium sulphate and evaporated in vacuo to give 2.60g of a near colourless oil.

The residue was purified by mplc using ethyl acetate/hexane (1:3) to give 2.55g of a near colourless oil (90%). Hplc analysis showed a majority isomer as a $C_{4,5}$ cis/trans pair as about 90% of the product and 10% minority isomer also as a $C_{4,5}$ cis/trans pair. Nmr analysis confirmed the majority 3-(R *-t-butyldimethylsilyloxyethyl) isomer: nmr in $CDCl_3$ 0.04 (d, 6H, 10% satellites); 0.84/0.88 (s/q, 12H); 1.18 (q/t, 3H); 2.02 (m, 1H); 2.88/2.94 (q/q, 5:4 ratio J = 5.3 and 3.0 each, 1H $C_{4,5}$ cis/trans isomers); [3.02/3.09 satellites, 10% 1H]; 3.26 (m, 2H); 3.66/3.72 (q/q, 5:4 ratio, J = 3.6, 3.0 and 5.3, 3.0 resp., 1H); 4.13 (m, 2H);
4.86 (d, 5:4 ratio, 2H); 4.53 (q, 1H); 7.26 (m, 10H); M + H (EI) = 468(weak), 410.

(d) 3S *-(1R *-t-butyldimethylsilyloxyethyl)-4R *-(2-hydroxy-1RS-methylethyl)trans-azetidin-2-one

The product of (c) above (1.88g) was stirred in a mixture of diethyl ether (20ml) and liquid ammonia (50ml) under a 'Dry Ice' condenser. Sodium metal (400mg) in small portions was added over about 30 minutes until a intense blue-green colouration persisted. After stirring for a further 30 mins, ammonium chloride (1.10g) was added and the ammonia allowed to evaporate under a stream of argon. The residue was treated with 30ml water and chloroform (300ml). The organic layer was filtered through anhydrous sodium sulphate and evaporated in vacuo to give 1.25g of a pale yellow oil. Tlc analysis revealed two distinct spots for the $C_{4,5}$ cis/trans isomer of Rf = (approx) 0.35/0.28 (silica gel /ethyl acetate). Purification by mplc using silica gel and chloroform/methanol (95:5) gave 780mg of the mixture of isomers as a near colourless oil (70%), nmr in $CDCl_3$ 0.10 (q, 6H); 0.90 (d, 9H); 0.88 (m, 3H); 1.30 (q, 3H); 1.82 (broad singlet, 1H); 2.85 (q, 1H); 3.13 (q, $\frac{1}{2}$ H); 3.30 (q, $\frac{1}{2}$ H); 3.50 (m, 2H); 3.69/3.75 (d/d, 1H); 4.04 (sextet, 1H); M + H (CI) = 288.

(e) 3S *-(1R *-t-butyldimethylsilyloxyethyl)-4S *-(1RS-carboxy-ethyl)-trans-azetidin-2-one

The product of (d) above (375mg) was stirred in 4ml pyridine at ambient temperature and a solution of 0.88g chromium trioxide in 0.6ml $H_2O$/8ml pyridine added in one portion. The mixture was stirred at ambient temperature for 20hrs. A further 0.50g chromium trioxide in 10ml pyridine (no $H_2O$) was then added and stirring continued for 5 hours.

The reaction was quenched by addition of excess sodium bisulphite and 50ml water and then the total reduced to low volume in vacuo to remove as much pyridine as possible. Ethyl acetate (200ml) and water (100ml) was added and aqueous pH adjusted to about 3 with dil. hydrochloric acid. Three further pH washes, water and saturated brine after filtration through anhydrous sodium sulphate and evaporation in vacuo gave 200mg crude product as a near colourless gum. Tlc analysis on silica gel in ethyl acetate: ethanol 9:1 showed the product as two isomers ($C_{4/5}$ cis/trans), at Rf's 0.34 and 0.45.

Purification by mplc on silica using ethyl acetate:ethanol (9:1) to remove 80ml uncharged starting material gave 40mg product as a low m.p. solid. Nmr ($CDCl_3$): 0.08 (q, 6H); 0.78 (d, 9H); 1.26 (m, 3H); 1.30 (q, 3H); 2.78/2.82 (m/m 1H total); 3.06 (m, 1H); 3.64/3.97 (m, 1H total, C4/5 isomers); 4.25 (q, 1H); 6.17/6.38 (m/m 1H total); M-H (negative FAB) 300.

The product of (e) above may be cyclised to yield a carbapenem of formula (I) by known methods for example as described in Heterocycles 21, 29 (1984); EP 126587 and Tetrahedron Letters 26, 4739 (1985).

Example C

(a) 3-Acetyl-1-benzyl-4-(2,2-diethoxyethyl) trans-azetedin-2-one.

To a suspension of pyridinium chlorochromate (2.2g) and sodium acetate (0.9g) in dichloromethane (40ml) was added a solution of the product of Example 5. The resulting mixture was stirred for 1h then diethyl ether (80ml) was added, and the mixture stirred for a further 30 min. The organic layer was decanted and the solid residue washed with diethyl ether (3 x 50ml). The combined organics were filtered through a pad of florisil and silica (50g each), then the solvent was evaporated to dryness. The residue was purified by chromatography on silica using ethyl acetate/hexane 1:1. The appropriate fractions were evaporated to dryness to give 500mg (31%) of the title compound as a pale yellow oil.

A sample had the following Nmr in deuterochloroform:

1.10 (m,6H); 1.72 (m,1H); 1.97 (m,1H); 2.28 (s,3H); 3.45 (m,4H); 4.00 (m,1H); 4.05 (d,1H); 4.20 (d,1H); 4.58 (d,1H); 4.40 (m,1H); 7.30 (m,5H).

M + H(FAB) = 320.

(b) 1-Benzyl-4-(2,2-diethoxyethyl)-3S *-(1R *-hydroxyethyl)-trans-azetedin-2-one.

To the product of (a) above (alternatively the product of Example 6 could be used) in diethylether (100ml) under argon was added powdered potasium iodide (1.2g). The mixture was stirred at ambient temperature for 30 min, then a solution of potassium tri-sec-butyl borohydride (1 molar) in tetrahydrofuran (120ml) was added dropwise. The resulting mixture was stirred at ambient temperature for 3h. A saturated solution of ammonium chloride (50ml) was added slowly, the organic layer removed and the aqueous extracted with diethyl ether. The organic layers were combined, extracted with saturated sodium chloride solution, dried over magnesium sulphate and evaporated to dryness. The residue was purified by chromatography on silica using ethyl acetate/hexane 1:1 increasing to ethyl acetate/hexane 3:1. The appropriate fractions evaporated to dryness gave 1.25g (81%) of the title compound as a pale yellow oil.

A sample had the following Nmr in deuterochloroform:

1.15 (m,6H); 1.30 (d,3H); 1.72 (m,1H); 2.02 (m,1H); 2.74 (s,1H); 2.95 (m,1H); 3.50 (m,5H); 4.08 (m,1H); 4.15 (d,1H); 4.61 (d,1H); 4.51 (m,1H); 7.28 (m,5H).

M + H(CI) = 322.

(c) 4-(2,2-diethoxyethyl)-3S *-(1R *-hydroxyethyl)trans-azetedin-2-one.

To the product of (b) above (1.0g) in a mixture of diethyl ether (5ml) and liquid ammonia (50ml) was added, in small partions, sodium (0.20g) until a blue colouration persisted. Ammonium chloride (1.0g) was added in portions and the ammonia allowed to evaporate. To the residue was added water (10ml) and the resulting mixture extracted with chloroform (3 x 25ml). The organic extracts were dried over magnesium sulphate and evaporated to dryness. The residue was purified by chromatography on silica using ethyl acetate/hexane 3:1 then ethyl acetate. The appropriate fractions evaporated to dryness gave 0.670g (93%) of the title compound as a pale yellow oil.

A sample had the following Nmr in deuterochloroform.

1.20 (m,6H); 1.3 (d,3H); 1.98 (m,2H); 2.64 (s,1H); 2.86 (m,1H); 3.60 (m,5H); 4.15 (m,1H); 4.62 (m,1H); 6.15 (s,1H).

M + H(CI) = 232, 2M + H(CI) = 463.

The product of (c) above may be cyclised to yield a carbapenem of formula (I) by known methods for example as described in heterocycles 12, 1183,1189,1301 (1979); J. Amer. Chem. Soc. 102, 2060 (1980).

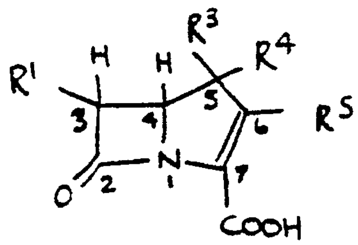 (I)

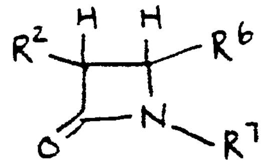 (II)

$R^2-C\equiv C-M$ (III)

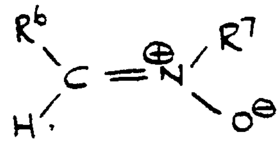 (IV)

$R^2-C\equiv CH$ (V)

$R^6CHO$ (VI)

$R^7NHOH$ (VII)

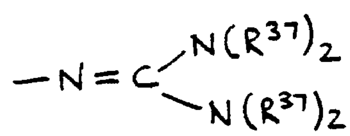 (IX)

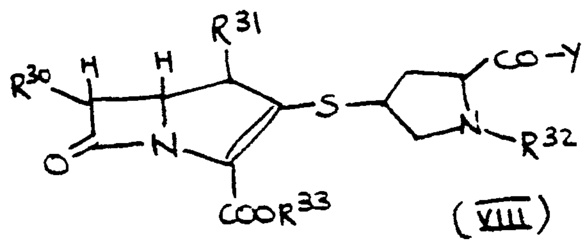

(VIII)

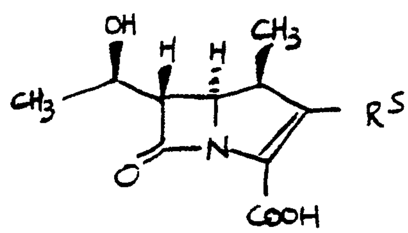

(Ia)

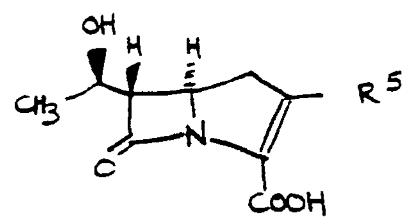

(Ib)

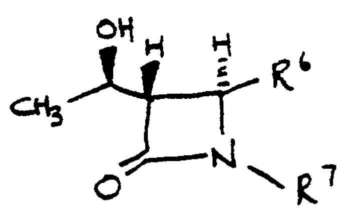 (IIa)

SCHEME A

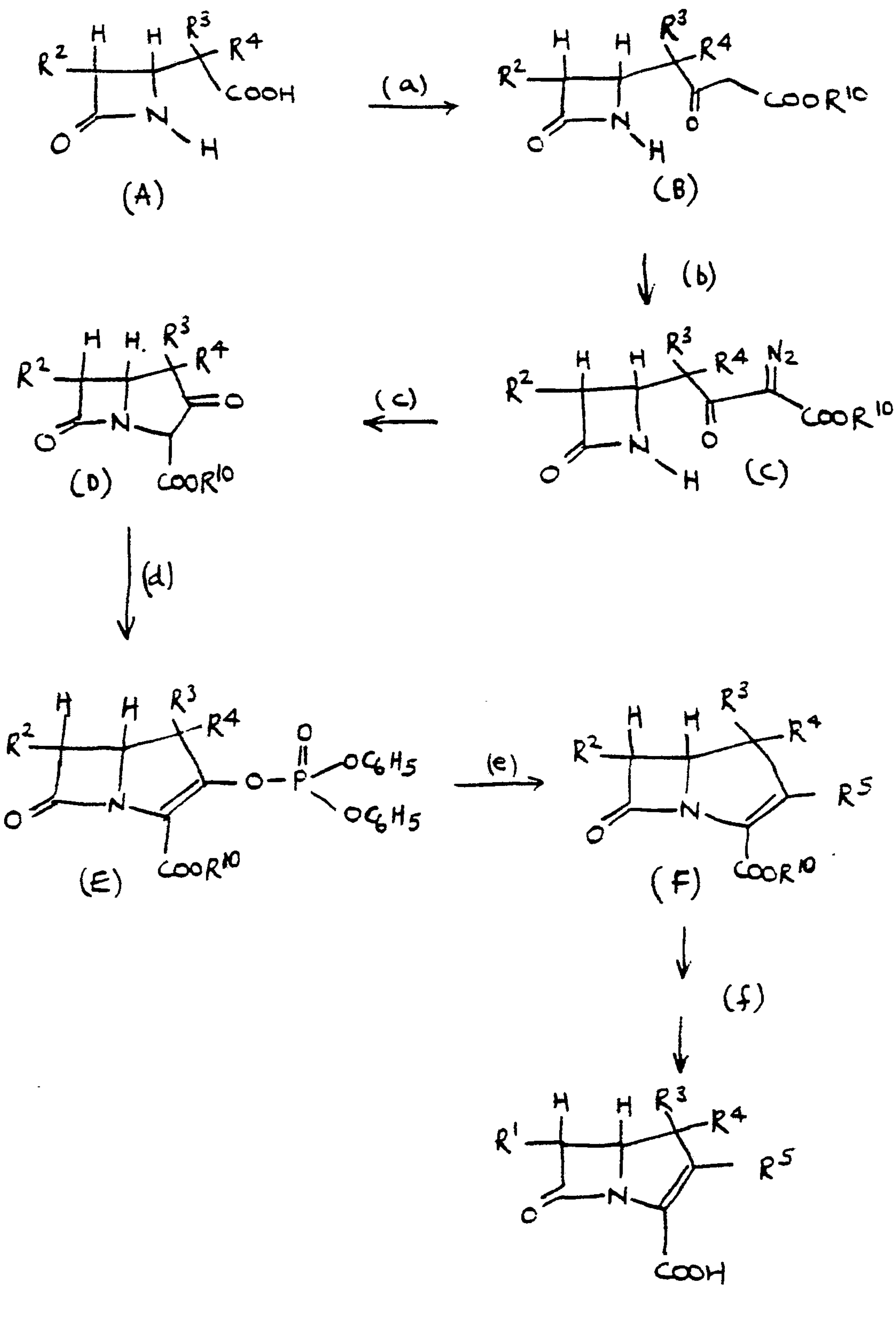


## Claims

1. A process for the manufacture of a $\beta$-lactam compound of the formula (II):

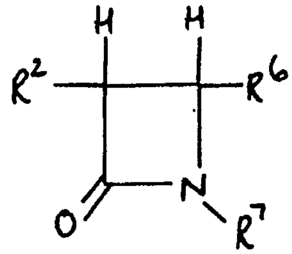

(II)

wherein $R^2$ is (1-4C)alkyl optionally substituted by one or more hydroxy groups, protected hydroxy groups or halogen atoms or $R^2$ is a group $R^2a$ which is a precursor of such a group wherein $R^2a$ is an acyl group or a protected form thereof or is an alkenyl group

$R^6$ is

(i) $-C(R^3)(R^4)X$ ;
(ii) $-C(R^3)(R^4)CH_2X$ ;
(iii) $-C(R^3)(R^4)COCH_2X$ ;
(iv) -COOH or -COSH ;
(v) $-C(R^3)(ZR^a)X$ ;

wherein $R^3$ and $R^4$ are independently hydrogen or (1-4C)alkyl, $R^a$ is (1-4C)alkyl or phenyl, X is selected from -COOH, -CHO and $-CH_2OH$ and Z is oxygen, sulphur or selenium (wherein in any of the above groups $R^6$ any carboxyl, thiocarboxyl, oxo or hydroxy groups may be protected); and $R^7$ represents hydrogen, an amino protecting group or a group $-C(R^3)(R^4)COOH$ or a protected form thereof:

which process includes the step of reacting a metal alkyne of the formula (III) with a nitrone of the formula (IV):

$R^2-C\equiv C-M$ (III)

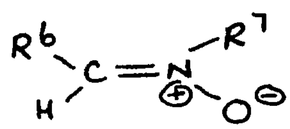

(IV)

wherein $R^2$, $R^6$ and $R^7$ are as hereinbefore defined and M is a metal ion.

**2.** A process according to claim 1 wherein $R^2$ is hydroxy(1-4C)alkyl wherein the hydroxy group is optionally protected by (1-4C)alkoxycarbonyl, halo(1-4C)alkoxycarbonyl, benzoyloxycarbonyl, p-methoxybenzoyloxycarbonyl, o-nitrobenzoyloxycarbonyl, p-nitrobenzoyloxycarbonyl, tri-(1-4C)alkylsilyl or benzyl.

**3.** A process according to either claim 1 or claim 2 wherein $R^2$ is a 1-hydroxyethyl, 1-(protected hydroxy)-ethyl, acetyl or ethenyl group.

**4.** A process according to any one of claims 1 to 3 wherein the metal alkyne is a copper (I) alkyne.

**5.** A process according to any one of claims 1 to 4 wherein $R^3$ and $R^4$ are independently hydrogen.

**6.** A process according to any one of claims 1 to 4 wherein one of $R^3$ and $R^4$ is methyl and the other is hydrogen.

**7.** A process according to any one of claims 1 to 6 wherein $R^7$ is benzyl.

**Revendications**

**1.** Procédé de production d'un composé de $\beta$-lactame de formule (II) :

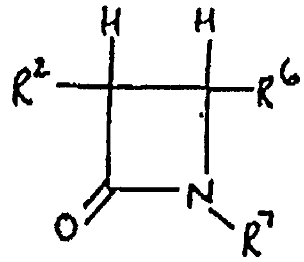

(II)

dans laquelle $R^2$ est un groupe alkyle en $C_1$ à $C_4$, facultativement substitué par un ou plusieurs groupes hydroxy, groupes hydroxy protégés ou atomes d'halogènes, ou bien $R^2$ est un groupe $R^2a$ qui est un précurseur de ce groupe, $R^2a$ étant un groupe acyle ou une forme protégée d'un groupe acyle ou un groupe alcényle,

$R^6$ représente

(i) $-C(R^3)(R^4)X$ ;

(ii) $-C(R^3)(R^4)CH_2X$ ;

(iii) $-C(R^3)(R^4)COCH_2X$ ;

(iv) -COOH ou -COSH ;

(v) $-C(R^3)(ZR^a)X$ ;

où $R^3$ et $R^4$ représentent, indépendamment, l'hydrogène ou un groupe alkyle en $C_1$ à $C_4$, $R^a$ est un groupe alkyle en $C_1$ à $C_4$ ou phényle, X est choisi entre -COOH, -CHO et $-CH_2OH$ et Z est l'oxygène, le soufre ou le sélénium (dans l'un quelconque des groupes $R^6$ ci-dessus, tout groupe carboxyle, thiocarboxyle, oxo ou hydroxy pouvant être protégé) ; et $R^7$ représente l'hydrogène, un groupe protégeant la fonction amino ou un groupe $-C(R^3)(R^4)COOH$ ou une forme protégée de ce groupe : procédé qui comprend l'étape de réaction d'un dérivé métallique d'alcyne de formule (III) avec une nitrone de formule (IV) :

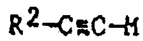

(11)

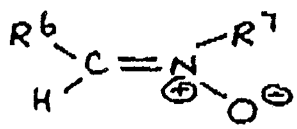

(IV)

où $R^2$, $R^6$ et $R^7$ ont les définitions données ci-dessus et M est un ion métallique.

2. Procédé suivant la revendication 1, dans lequel $R^2$ est un groupe hydroxy(alkyle en $C_1$ à $C_4$), dans lequel le groupe hydroxy est facultativement protégé par un groupe (alkoxy en $C_1$ à $C_4$)carbonyle, (halogénalkoxy en $C_1$ à $C_4$)carbonyle, benzoyloxycarbonyle, p-méthoxybenzoyloxycarbonyle, o-nitrobenzoyloxycarbonyle, p-nitrobenzoyloxycarbonyle, tri-(alkyle en $C_1$ à $C_4$)silyle ou benzyle.

3. Procédé suivant la revendication 1 ou la revendication 2, dans lequel $R^2$ est un groupe 1-hydroxyéthyle, 1-(hydroxy protégé)éthyle, acétyle ou éthényle.

4. Procédé suivant l'une quelconque des revendications 1 à 3, dans lequel le dérivé métallique d'alcyne est un dérivé d'alcyne de cuivre (I).

5. Procédé suivant l'une quelconque des revendications 1 à 4, dans lequel $R^3$ et $R^4$ représentent indépendamment l'hydrogène.

6. Procédé suivant l'une quelconque des revendications 1 à 4, dans lequel l'un de $R^3$ et $R^4$ est un groupe méthyle et l'autre est l'hydrogène.

7. Procédé suivant l'une quelconque des revendications 1 à 6, dans lequel $R^7$ est un groupe benzyle.

## Ansprüche

1. Verfahren zur Herstellung einer $\beta$-Lactam-Verbindung der Formel (II)

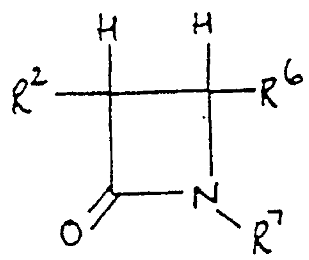

(II)

worin $R^2$ für (1-4C)Alkyl, das gegebenenfalls durch ein oder mehrere Hydroxygruppen, geschützte Hydroxygruppen oder Halogenatomen substituiert ist, oder für eine Gruppe $R^2a$ steht, die ein Vorläufer einer solchen Gruppe ist, worin $R^2a$ eine Acylgruppe oder eine geschützte Form davon oder eine Alkenylgruppe ist;
$R^6$ für
(i) $-C(R^3)(R^4)X$ ,
(ii) $-C(R^3)(R^4)CH_2X$ ,
(iii) $-C(R^3)(R^4)COCH_2X$ ,
(iv) -COOH oder -COSH oder
(v) $-C(R^3)(ZR^a)X$
steht, worin $R^3$ und $R^4$ unabhängig für Wasserstoff oder (1-4C)Alkyl stehen, $R^a$ für (1-4C)Alkyl oder Phenyl steht, X ausgewählt ist aus -COOH, -CHO und $-CH_2OH$, und Z für Sauerstoff, Schwefel oder Selen steht (wobei in allen obigen Gruppen $R^6$ jegliche Carboxyl-, Thiocarboxyl-, Oxo- oder Hydroxy-Gruppe geschützt sein kann); und
$R^7$ für Wasserstoff, eine Aminoschutzgruppe oder eine Gruppe $-C(R^3)(R^4)COOH$ oder eine geschützte Form davon steht;
bei welchem Verfahren ein Metallalkin der Formel (III) mit einem Nitron der Formel (IV)

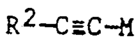 (III) 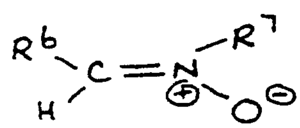 (IV)

worin $R^2$, $R^6$ und $R^7$ die oben angegebenen Bedeutungen besitzen und M für ein Metallion steht, umgesetzt wird.

2. Verfahren nach Anspruch 1, bei welchem $R^2$ für Hydroxy(1-4C)alkyl steht, worin die Hydroxygruppe gegebenenfalls durch (1-4C)Alkoxycarbonyl, Halogeno(1-4C)alkoxycarbonyl, Benzoyloxycarbonyl, p-Methoxybenzoyloxycarbonyl, o-Nitrobenzoyloxycarbonyl, p-Nitrobenzoyloxycarbonyl, Tri(1-4C)alkylsilyl oder Benzyl geschützt ist.

3. Verfahren nach Anspruch 1 oder 2, bei welchem $R^2$ für eine 1-Hydroxyethyl-, 1-(geschützte Hydroxy)-ethyl-, Acetyl- oder Ethenylgruppe steht.

4. Verfahren nach einem der Ansprüche 1 bis 3, bei welchem das Metallalkin aus Kupfer-(I)-alkin besteht.

5. Verfahren nach einem der Ansprüche 1 bis 4, bei welchem $R^3$ und $R^4$ unabhängig für Wasserstoff stehen.

6. Verfahren nach einem der Ansprüche 1 bis 4, bei welchem eines der Symbole $R^3$ und $R^4$ für Methyl und das andere für Wasserstoff steht.

7. Verfahren nach einem der Ansprüche 1 bis 6, bei welchem $R^7$ für Benzyl steht.